# EUROPEAN PATENT APPLICATION

(11) **EP 3 539 584 A1**
(43) Date of publication of application: **18.09.2019**
(21) Application number: 17870134.8
(22) Date of filing: 09.11.2017
(51) Int. Cl.: A61M 1/10, A61M 1/12, F04D 3/02

(54) **MICROAXIAL PUMP FOR ASSISTING BLOOD CIRCULATION (VARIANTS)**

(30) Priority: 09.11.2016 RU 2016144052
(71) Applicant: Koroteev, Alexey Vasilievich, Moscow 125284 (RU); Banin, Evgeny Petrovich, Pos. Dubna, Tulskaya obl. 301160 (RU); Barakov, Vladimir Nikolaevich, Ufa 450006 (RU)
(72) Inventor: Koroteev, Alexey Vasilievich, Moscow 125284 (RU); Banin, Evgeny Petrovich, Pos. Dubna, Tulskaya obl. 301160 (RU); Barakov, Vladimir Nikolaevich, Ufa 450006 (RU)
(74) Representative: Chimini, Francesco
(86) International application number: PCT/RU2017/000840
(87) International publication number: WO 2018/088939

(57) **Abstract**

The group of inventions relates to medical technology, and more particularly to variants of a device for pumping blood having a noncontact magnetic coupling. In a first variant, the device comprises a multistage pump unit. The pump unit comprises a housing and a spacer shaft. Two axial stages containing impellers with curved vanes, and a stator are disposed on the spacer shaft. The device comprises a power unit with a hermetic housing, a motor, and a motor-driven main shaft. The device for pumping blood also comprises a magnetic coupling consisting of an external driving half-coupling on the main shaft of the power unit and an internal driven half-coupling on the end surface of the mount of the impeller of the second axial stage of the pump unit. The power unit transmits torque to the spacer shaft of the pump unit from the motor. In a second variant, the power unit is situated outside the patient's body and comprises a flexible transmitting shaft situated inside the patient's body. Terminal magnetic half-couplings are rigidly fastened to the ends of the flexible shaft. The first terminal half-coupling contacts with the internal driven half-coupling of the pump unit. The second terminal half-coupling contacts with the external driving half-coupling of the power unit. The technical effect is a reduction in blood trauma, a reduction in the rate of rotation of the impellers, and a reduction in the overall dimensions of the device.

## Description

### FIELD

This group of inventions relate to medical equipment in general, and in particular, to devices for pumping single-phase or multi-phase fluids without altering their properties (e.g. pump-assisted blood circulation at various surgical interventions) and may be used as an auxiliary device with cardiac decompensation or as a principal pump in systems of assisted blood circulation.

### BACKGROUND

Pumping sensitive polycyclic liquids (e.g. suspensions) under various factors (e.g. shearing stress, high temperatures) may cause irreversible changes in the structure and composition of the work fluid.

Blood is a polydisperse system (a suspension of formative elements (erythrocytes, thrombocytes, leucocytes) in the plasm). The blood is permanently circulating in the body within a closed system that prevents its interaction with environment and, hence, preserves its properties.

Extensive research established that contacts with alien materials and other interactions (e.g. mechanical) may cause destruction of blood erythrocytes and discharge hemoglobin into blood as well as activate thrombocytes (which leads to clotting). Such interaction with alien materials and side effects may cause serious complications. Resulting trombus may appear on extraneous surfaces, create local resistance to the blood flow, break off and clog up small vessels. A raised physiological level of hematolysis impacts functioning of various organs and, in critical cases, may lead to their damage.

In the last decades, the principal cause of mortality is attributed to cardiovascular disease. If there is a need to replace a heart or support heart function, a patient may require transplantation of a donor organ with a limited resource. The latest research accumulates more and more evidence in favor of opportunities to support the heart's pumping function with artificial systems (such as an artificial heart or a ventricle). As for today, such devices cannot compete with donor organs in guaranteed longevity but progress in this area may soon merit appreciation.

The existing technical level makes us believe that development of assisted blood circulation is focused on miniature implanted pumps that ensure physiological blood flow with minimal surgical intrusion. Such pumps are designed on the basis of a given physiological pressure difference and heart flow parameters. The miniature pumps fall into three classes: axial pumps, centrifugal pumps, and diagonal pumps. As a rule, the axial and diagonal pumps are blood support devices of smaller dimensions but, with equal starting parameters, a centrifugal pump provides required flow at lower rotation velocity of movable parts, thus, resulting in less blood trauma.

The design of used miniature axial pumps typically consists of a cylinder tube with an incorporated impeller and support elements. The requirement for smaller dimensions and, in particular, implantability via the cardiovascular system leads to an extremely high speed of the impeller in order to reach physiological parameters of blood circulation. High rotation velocity inevitably leads to higher blood trauma and limits both the motor resource and its useful duration inside the body. As a rule, such pumps are used for short periods (up to 15 days) in critical invasions of limited scale surgery. Devices of this type are introduced in the body via large blood vessels and exclude transthoracic surgery.

There is an axial vane pump for assisted blood circulation (RU2131271 C1, published on 10.06.1999) that has an impeller with vanes inside housing, rotated by a flexible shaft set on bearings press-fitted inside the bushing of straightener housing. A similar design is used in another invention (EP1207934 B1, published on 29.05.2002). This represents an intravascular blood pump with a flexible shaft and an motor placed outside the patient's body. A distinguishing feature of the two designs is the need for lubrication to exclude blood getting between stationary and revolving elements of the pump and, even more importantly, to prevent blood getting into bearings and jamming them. During operations, such pumps are introduced via large vessels in the body (such as the vein below the groin) and conducted to a ventricle by a flexible guide. The size of vessels poses significant dimensional restraints on the device. For various patients the device needs to be less than 10 mm in diameter. An option with a flexible main shaft does not require miniature motors inside the body and permits a simpler design. However, a complex vessel configuration (e.g. in the curved area between the descending aorta and the aortic valve) using a flexible shaft poses restrictions on motor operation modes as there is a risk of shaft jamming due to its length and friction against the walls. Little consideration is given to the fact that the required physiological flow of 5 l/min through a cross section of up to 10 mm diameter is possible only at high revolutions (over 30 000 rev/min). High rotation velocity of the impeller leads to higher blood trauma, as rotation velocity directly affects shearing stress inside the pump flow.

At the technical level, there is also an axial vane pump for assisted blood circulation (RU2051695 C1, published on 10.01.1996), which is a development of invention (RU2131271 C1, published on 10.06.1999). This design has structural problems with the flexible shaft, in particular its low capacity in the required range of discharge. The impeller rotation of 15-20 thousand rpm allows discharge of up to 3.5 l/min, which is inadequate for replacing the heart function.

There is an invention (US5507629 A, published on 16.04.1996) that describes a pump with an impeller drive inside a ventricle. The pump is installed via the top of the left ventricle avoiding the curved area between the descending aorta and the aortic valve. Thus, it is placed between the top of the ventricle and the aortic valve. This pump design serves to redistribute flows as the blood may not only flow forward through the aortal valve but also backward (through the top of the ventricle directly to the descending aorta below the left ventricle). The pump is made up of a number of magnetic elements, which make its manufacture rather difficult. Another consideration is the necessity of a major surgical intervention.

The invention (US20120029265 A1, published on 02.02.2012) is a pump with one or several stators. With the help of a guide the pump is introduced through larger vessels into the ventricle cavity. It is positioned and fixed by way of the stent with shape memory. The motor coils are place directly inside the ventricle or the ascending aorta making it possible to do without a flexible shaft on account of a more complex design. The principle drawbacks of this design are the need for high velocity of the impeller (from 20 000 to 60 000 rpm) in order to supply the necessary physiological flow and small gaps between the housing and the rotor (about 0.1 mm). These factors contribute to a significant hematolysis on account of high shearing stress inside the pump flow channel.

A closely analogous device is the one for assisted blood circulation (US6176822 B1, published on 23.01.2001) that represents a miniature pump unit with a tiny electrical motor. The motor shaft has a cantilever hold on one side (proximal). On the other, distal side the motor shaft goes through a sleeve bearing and a seal ring. The motor shaft terminates with an impeller. The driving part is located above the aortal valve. The pump is introduced in the ventricle cavity by a flexible guide through larger vessels.

The disadvantages of this invention are:
- High speed of rotor;
- Single-sided shaft support requires an extremely robust bearing;
- The seal ring suffers wear from the high speed of rotation and a single-sided shaft support may lead to motor jamming due to high content of salts and other inclusions in the blood plasm.

### SUMMARY

This group of inventions serves to resolve the technical problem arising from considerably changed parameters of pumped single-phased and multi-phased fluids (e.g. blood) including blood trauma, stagnation and recirculation zones.

The technical effect of at least one embodiment of the present technology as disclosed herein is an reduction of blood trauma through lower perturbance in the flow inside the pump channel, lower velocity of impeller rotation, no need for local lubrication, and exclusion of power unit motor jamming.

The additional technical effect of at least one embodiment of the present technology as disclosed herein is intact skin (avoidance of invasion).

To achieve this technical effect, we propose the following design embodiment s for the blood pump with a noncontact magnetic coupling.

In accordance with at least one embodiment, a blood pumping device as described herein comprises a multi-stage pump unit and a power unit. The multi-stage pump unit having: a housing, a spacer shaft, at least two axial stages containing curved vane impellers placed on the spacer shaft inside the housing and containing a stator placed on the spacer shaft inside the housing. The power unit having: a hermetic housing, a motor, located inside said housing, and a motor-driven main shaft.

The impeller of the multi-stage pump unit is connected to the main shaft via a noncontact magnetic coupling, the noncontact magnetic coupling having: an external driving half coupling set on the main shaft and an internal driven half coupling located of an end mount of the impeller of the second axial - stage of the multi-stage pump unit.

In at least one embodiment, the stator has at least three straighteners and one of them being an inflow straightener.

In at least one embodiment, the vanes of the inflow straightener are aligned along an axis of the spacer shaft and/or at an angle to said axis.

In at least one embodiment, the number of curved vanes of at least one impeller is different from the number of vanes of at least one straightener of the stator.

In accordance with at least one embodiment a blood pumping device as described herein comprises a multi-stage pump unit and a power unit. The multi-stage pump unit includes at least two axial stages located on a spacer shaft inside a multi-stage pump unit housing, at least two axial stages containing impellers having curved vanes, and a stator. The power unit, which is located outside the patient's body, consists of a housing, an motor and a motor-driven main shaft. The main shaft transmits the torque to the spacer shaft of the pump unit by a flexible transmission shaft and a magnetic coupling via a magnetic field. The magnetic coupling having an external driving half coupling set on the main shaft of the power unit, and an internal driven half coupling located of an end side of a mount of the impeller of the second axial stage of the multi-stage pump unit.

The flexible transmission shaft is located inside the patient's body with a terminal magnetic half couplings fixed on each end of it. The first terminal half coupling has a magnetic contact with the internal driven half coupling of the pump unit and the second terminal half coupling has a magnetic contact with the external driving half coupling of the power unit.

In both variants of embodiment in some implementation options, the spacer shaft is located into at least one pass -through and at least one terminal bearing support.

In both variants of embodiment in some implementation options, sleeve bearings and/or roller bearings are used as bearings.

In both variants of embodiment in some implementation options, the curved vanes of the impellers of the multi-stage pump unit have the same or different profiles.

In at least one embodiment, , the flexible transmission shaft is supported by sleeve and/or roller bearings at equal or varied intervals along the shaft axis, the flexible transmission shaft is located inside the hollow flexible broach.

In at least one embodiment, the flexible shaft made in the form of a steel string with a round cross section.

In both variants of embodiment, the external driving half coupling is in the form of a pulley with permanent magnets integrated inside it and the internal driven half coupling contains permanent magnets integrated in the mount of impeller of the second axial -stage of the multi-stage pump unit.

In at least one embodiment, the first terminal half coupling is in the form of a pulley with permanent magnets and the second terminal half coupling is in the form of a bushing with permanent magnets integrated in its mount.

In at least one embodiment, the pulley of the first terminal half coupling and the bushing of the second terminal half coupling are located in respective hermetic housings with an adaptor cover rigidly connected to the hollow a flexible broach.

In both variants of embodiment in some implementation options, the external diameter of the housing of the multi-stage pump unit is smaller than the diameter of large blood vessels.

Lower rotation velocity of impellers is possible due to the use of at least two stages in the design of the axial pump.

The use of two stages of the axial pump serves to reduce motor velocity that leads to smaller shearing stress in the gaps between the pump housing and the impellers, thus, benefitting the flow of essential blood elements (erythrocytes, thrombocytes, leucocytes) through the pump channel. In order to support normal physiological parameters of cardiac function (capacity of 5-6 l/min, pressure difference up to 140 - 150 mm of mercury), a two-stage pump needs to produce up to 15 000 - 18 000 rpm.

To raise the pump efficiency, a straightener is put inside its entrance for better flow attraction onto the impeller vanes. A vane profile for the straightener is selected on the basis of the flow behavior at the entrance to the axial pump.

Installation of the power unit in a hermetic housing and its separation from the inside flow channel (the pump unit of the proposed device) with rotation transmitted to the impellers by the magnetic coupling prevents blood from getting into the motor and jamming it. These features contribute to longer operation of the microaxial pump.

The multi-stage pump is designed in such a way so that the number of curved vanes of at least one impeller differs from the number of vanes of at least one straightener of the stator. This serves for reduction of flow disturbance inside the pump channel and less blood trauma as compared to a design with divisible or equal numbers of impeller vanes and stator elements.

The distinguishing feature of the blood pumping device having a pump unit inside the patient's body and the power unit outside it, is that it fully excludes environment particles (blood) getting onto the friction surfaces: sleeve bearing - flexible shaft. This preserves sterility and reduces mechanical blood damage. The rotating torque is communicated bypassing the skin and does not change the environment (blood).

The perfectly hermetic noncontact flexible transmission preserves the sterile location of the pump inside the ventricle cavity and excludes the necessity to place in the body such power unit elements as stator coil. This embodiment of the invention provides an opportunity of replacing the power unit without re-implantation (in cases of wear or failure of the motor). This prolongs the useful life of pump operation, reduces the weight and volume of the transplant and excludes thermal effect on blood from heated stator coils.

### BRIEF DESCRIPTION OF THE DRAWINGS

The drawings included in the description of the invention are its integral part and serve to illustrate variants of its implementation. Together with the general description above and the detailed description of options that follows, they serve for clarification of the principles of the invention. Similar positions are used for denoting similar parts in drawings.
Figure 1 is a general view of the microaxial pump for the assisted blood circulation (the housing of the flow-through part with the second-stage straightener is dismantled).
Figure 2 is a general view of the microaxial pump for the assisted blood circulation with the motor and a housing of the flow-through part with the straightener of the second axial stage of the multi-stage pump unit.
Figure 3 is main parts and elements of the microaxial pump for the assisted blood circulation with an internal power unit.
Figure 4 is a general view of the microaxial pump for the assisted blood circulation with an internal power unit.
Figure 5 is main parts and elements of the microaxial pump for the assisted blood circulation with an external power unit.
Figure 6 is the external power unit and the flexible broach with terminal magnetic half couplings.
Figure 7 is the external power unit and one of terminal magnetic half couplings fixed on the flexible broach.
Figure 8 is an installation of the microaxial pump for the assisted blood circulation with the external power unit via the thigh artery and the ascending aorta.

### LEGEND

1 - Broach
2 - Power unit hermetic housing
3 - Second axial-stage impeller
4 - First axial stage straightener
5 - First- axial stage impeller
6 - Inflow straightener
7 - Flexible insert
8 - Terminal blood inlet
9 - Motor
10 - Magnetic half coupling
11 - Second axial-stage straightener
110 - Housing of flow-through part
12 - Power unit cover
13 - Cylindrical part of power unit housing
14 - Bushing on the motor shaft
15 - Bearing support, Sleeve/roller bearing
16 - Magnet
17 - Spacer shaft
18 - First terminal half coupling
180 - Second terminal half coupling
19 - Cover plate of magnetic half coupling on flexible shaft
20 - Housing of magnetic half coupling on flexible shaft
21 - Eyes for attaching housing of external power unit to skin
22 - Flexible transmitting shaft
23 - Housing of second magnetic half coupling
24 - Cover plate of power unit
25 - Housing of power unit
26 - Pulley of magnetic half coupling of motor
27 - Pulley of magnetic half coupling on flexible shaft
28 - Adaptor cover of housing of magnetic half coupling to flexible transmitting shaft
29 - Pump of assisted blood circulation with external power unit
30 - Direction of blood flow
31 - Skin

### DETAILED DESCRIPTION OF THE NON-LIMITING EMBODIMENTS

In general, the current invention belongs to the group of axial pumps for blood circulation and may be used as an ancillary device with deficient heart function or as the principal pump in systems of assisted blood circulation.

This microaxial pump belongs to the group of axial pumps based on power interaction between the vane and the flow around it. In axial pumps the flow of the liquid is parallel to the rotation axis of the impeller and does not change its direction after going through the pump channel.

A preferable option for such pumps is to use the principle of magnetic coupling to partition the blood side from the drive. This allows maximum restriction of blood contacting surfaces. A compact design facilitates its insertion into larger vessels.

A microaxial pump with a magnetic coupling for blood circulation contains inside its housing a power unit and a multi-stage pump unit with sequential impellers with curved vanes and a stator on a spacer shaft. The pump unit is connected to a blood inlet.

The power unit is made with opportunity to transmit the rotating torque to the spacer shaft of the pump unit.

When the pump unit consists of two axial stages, the each package of axial stages includes impeller of the first axial -stage or impeller of the second axial -stage with curved vanes and a stator. The impellers are press fitted on the spacer shaft or hermetically glued inside the housing of the pump unit in the form of a bushing.

The stator at the entrance to the pump includes at least one inflow straightener of the incoming flow with a calculated profile of vanes and at least two straighteners with a calculated profile of vanes. The stator employs direct radial vanes in the inflow straightener of the incoming flow in order to ensure axial admission of the work fluid to the next axial stage impeller.

The stator is set on the spacer shaft with the help of at least one open-type and at least one terminal bearing, which may be of sleeve and/or roller types.

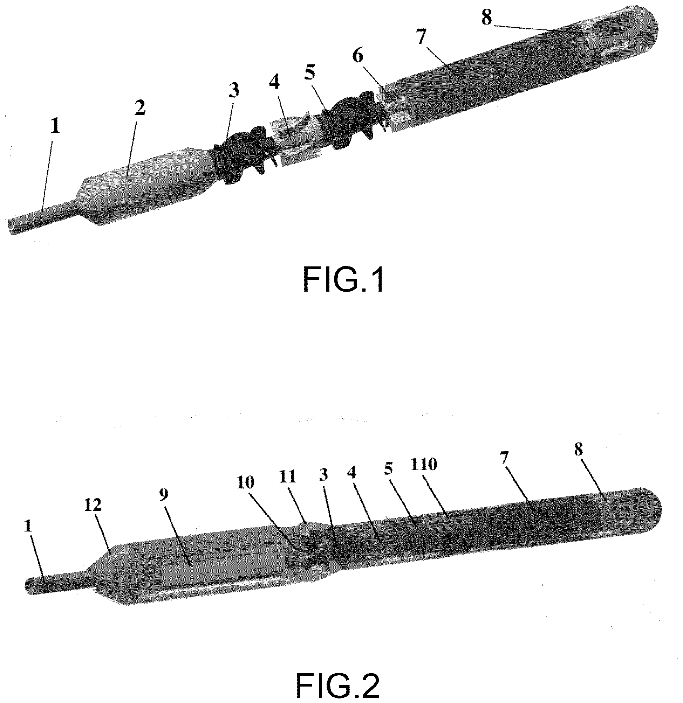

Referring to Figures 1, in accordance with the non-limiting embodiments of the present technology, the blood pumping device with the noncontact magnetic coupling (the microaxial pump with the noncontact magnetic coupling for blood circulation is the same). The blood pumping device comprises a multi-stage pump unit and a power unit with hermetic housing 2 and a main shaft. The hermetic housing 2 of the power unit via cover 12 of the power unit (figure 2) connects to a broach 1, which is a hollow tube.

The housing 2 of power unit is adjacent to an impeller 3 of the second axial stage of the multi-stage pump unit. The second axial stage impeller 3 is either press fitted or glued on a spacer shaft 17 (not shown on figure 1). The spacer shaft 17 passes through a first axial stage stationary straightener 4 that contains bearing support 15 with sleeve or roller bearings. Behind the first axial stage straightener 4 there is first axial stage impeller 5. The first axial stage impeller 5 and the second axial stage impeller 3 are fitted similarly on the spacer shaft 17. Behind the first axial stage impeller 5 there is a stationary inflow straightener 6 with bearing support 15. All the implementation variants may employ either roller or sleeve bearings. Behind the inflow straightener 6 there is a flexible insert 7, representing a flexible hollow thin-walled tube. The flexible insert 7 has a terminal blood inlet 8 press fitted or glued on it. The terminal blood inlet 8 is the stiff tube-like element perforated on edge with a distal rounded tip. Referring to Figures 1, a housing of a flow-through part with a second axial stage straightener is dismantled.

The general view of the microaxial pump with the noncontact magnetic coupling for blood circulation with an motor and housing of the flow-through part with the second-stage straightener is represented in Figure 2.

The blood inlet that takes in the fluid has the form of hollow cylindrical flexible insert 7. It connects to terminal blood inlet 8 in the form of a stiff tube perforated on its edge with a distal rounded tip, a two-stage pump unit and a power unit.

The terminal blood inlet 8 is connected to a housing of a flow-through part 110 by means of the flexible insert 7 attached to external surface or internal surface of the housing of the flow-through part 110. The flexible insert 7 is glued or soldered to the housing of the flow-through part 110. A second axial stage straightener 11 is glued, soldered or press fitted to the housing of the flow-through part 110 on its external surface or internal surface. The second axailstage straightener 11 constitutes a hollow, thin-walled tube of variable diameter or constant diameter aligned with pump axis with exit holes for fluid. Further on, the straightener 11 of the second axial stage is press fitted or otherwise connected to the housing 2 of the power unit. The power unit contains a motor 9 and a magnetic half coupling 10 transmitting rotation torque from the motor 9 by way of magnetic field on to the second axial stage impeller 3 and the first axial stage impeller 5. The first axial stage impeller 5 and the second axial stage impeller 3 are located on the spacer shaft 17 (not shown on figure 2). Stationary support for the spacer shaft 17 is provided by the first axial stage straightener 4 with sleeve or roller bearings and by the inflow straightener 6 with sleeve or roller bearings. The first axial stage straightener 4 and the inflow straightener 6 are press fitted, glued or soldered to internal wall of the housing of the flow-through part 110, resulting as stationary (immovable) elements.

Conducting broach 1 is attached to a motor 9 housing by means of the power unit cover 12, which is press fitted, glued or soldered into power unit housing 2.

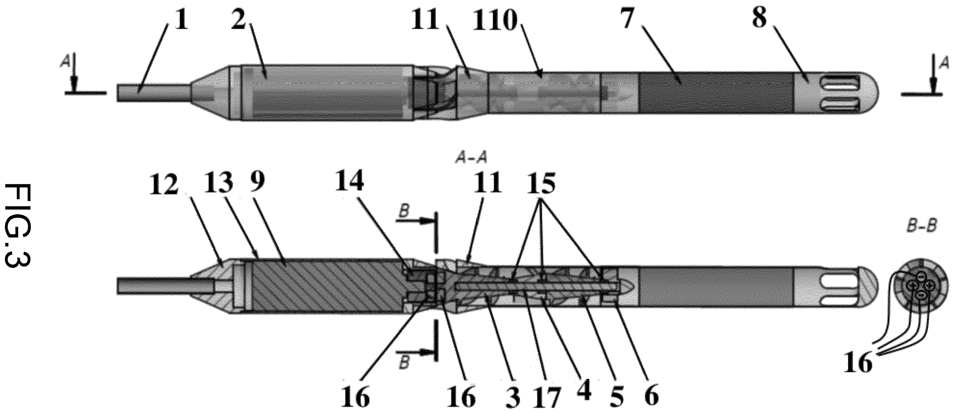

Work fluid comes in through the terminal blood inlet 8 and passes via the flexible insert 7 connected to the housing of flow-through part 110 (figure 3). The flow comes to the inflow straightener 6 and gets straightened. Thanks to the vanes of the inflow straightener 6 the circumferential component of speed gets reduced and the flow itself becomes almost axial. After the inflow straightener 6 the flow comes to vanes of the first axialstage impeller 5 that imparts rotation torque to the flow and pushes it onto stationary vanes of the first axial stage straightener 4, where the kinetic energy of the flow is transformed by braking against the immovable vanes into pressure energy. Then the flow comes through the second axial stage impeller 3 where it gets additional kinetic energy again from rotation that is transformed in pressure energy when passing through the holes of the second axial stage straightener 11.

The motor 9 is located in a cylinder part of power unit housing 13. A bushing 14 with magnets 16 is set on the main shaft to form magnetic half coupling 10 (figure 2). Farther on, rotating torque is transmitted via the magnetic field formed between the magnets 16 of bushing 14 and the magnets in the mount of second axial stage impeller 3 located on common spacer shaft 17 (figure 3). The first- and second- stage impellers are on common spacer shaft 17. The torque transmitted to the second axial stage impeller 3 is also transmitted to the first-axial stage impeller 5. The spacer shaft 17 is fixed in roller and sleeve bearings in stationary first axial stage straightener 4 and inflow straightener 6. The number of magnets in bushing 14 and the second axial stage impeller is not less than four and the magnet poles alternate.

In the alternative embodiment of the device for blood circulation, the number of curved vanes of at least one impeller differs from the number of vanes of at least one straightener of the stator that helps reduce blood trauma.

The vane profiles of first axial stage 5 and second axial stage 3 impellers in both embodiments are determined by mathematic models based on physiological parameters of a healthy heart; discharge of up to 5-6 l/min and pressure difference of up to 140 - 150 mm of mercury. The curved vanes of first axial stage 3 and second axial stage 5 impellers may have the same and/or different profiles.

The vane profile of the inflow straightener 6 is selected based on the flow behavior at the entrance to the pump unit. Straightener vanes of first 4 and second 11 stages may be set in parallel to the axis passing through the main shaft and at an angle to it. The straightener vanes of first 4 and second 11 stages have a complex design profile to ensure flow constriction and pressure boost.

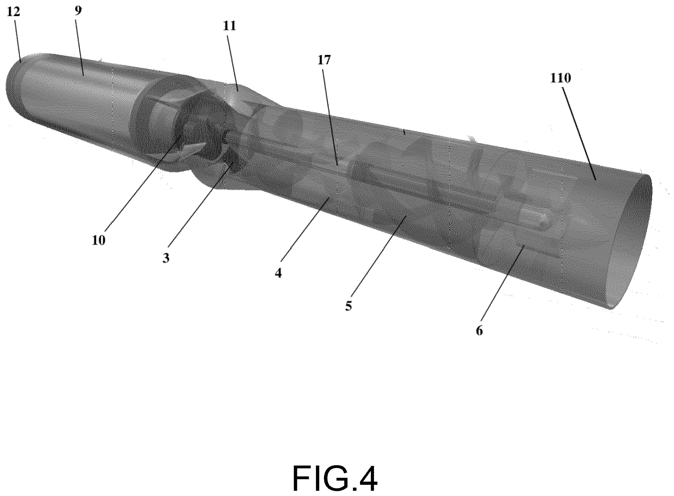

The noncontact magnetic coupling of the microaxial pump as seen in figures 3 and 4 consists of an internal driven half coupling a hermetic cover separating the pump part from the power unit, and an external driving half coupling located in the power unit. The internal driven half coupling is made of permanent magnets 16 located on the end surface of the mount of second axial stage impeller 3 of the pump unit. The half couplings may be multi-pole with alternating poles.

Permanent magnets 16, as seen in the sectional view B-B of figure 3, are press fitted or glued to the mount of second axial stage impeller 3, and isolated by the hermetic cover, which, in its turn, is glued or soldered to the end surface of the mount of second axial stage impeller 3. The mount of second axial stage impeller 3 has at least four permanent magnets at equal angle increment from each other.

In one variant of implementation the power unit is internal and placed inside the patient's body. This variant of implementation is shown in figure 1-4. To separate the power unit from 'the blood zone' it is placed in a hermetic housing directly inside a large vessel (e.g. aorta) and includes motor 9 with a main shaft in the hermetic housing 2.

The external driving half coupling in the form of bushing 14 with permanent magnets 16 is set directly on main shaft 9. Permanent magnets 16 are either press fitted or glued to the mount of bushing 14.

The rotating main shaft of the motor 9 puts in motion the external driving half coupling set on the main shaft and transmits the rotation torque through hermetic housing 2 on to the driven half coupling comprising magnets in the mount of second axial stage impeller 3 via the permanent magnetic field.

Cover 12 of the hermetic housing 2 of the power unit has a conical shape and an exit port. It is set on hermetic housing 12 usually by screwing.

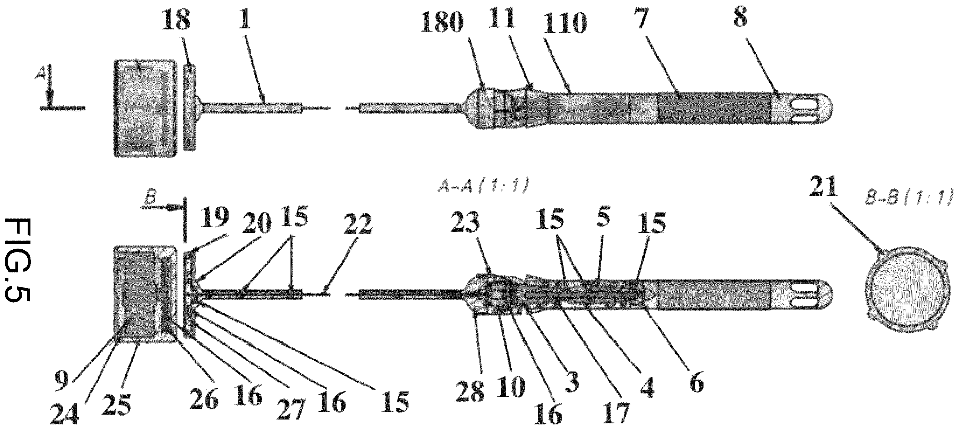

In another embodiment, the power unit is external and placed outside the patient's body. The power unit is installed inside a housing 25 (figure 5) outside the body. It comprises motor 9 with cover 24 and the main shaft.

The main shaft is equipped with pulley 25 of the external driving half coupling of the power unit with permanent magnets 16. The torque from main shaft 9 is also transmitted to the external driving half coupling.

The device has additional flexible transmitting broach 1 with terminal half couplings, one of which has a magnetic contact with the internal driven half coupling comprising magnets 16 in the mount of second axial stage impeller 3 in the pump unit, while another - with the external driving half coupling of the power unit consisting of pulley 26 with magnets 16. The terminal half couplings are press fitted/glued on a flexible shaft 22 that passes inside the hollow flexible broach 1 through the sleeve/roller bearings set at constant or variable intervals along the length of flexible transmitting shaft 22. In one of the variants flexible transmitting shaft 22 made in the form of a steel rod of round cross section.

One of the terminal half couplings 18 is set in motion via a permanent magnetic field from the external magnetic half coupling consisting of pulley 26 and magnets 16 set on the main shaft of external motor 9 connected to the control board that regulates the modes of pump operation in the physiological (for the patient) discharge range. The control board may be installed on the patient or on a console next to the patient. Operation modes are selected depending on a personal requirement for oxygen. Thus, higher oxygen consumption requires faster motor rotation that provides higher discharge.

Pulley 27 of terminal half coupling 18 with permanent magnets 16 is set on flexible transmitting shaft 22 by means of sleeve/roller bearing 15 inside housing 20 with cover 19 tightly connected to broach 1 by glue or soldering.

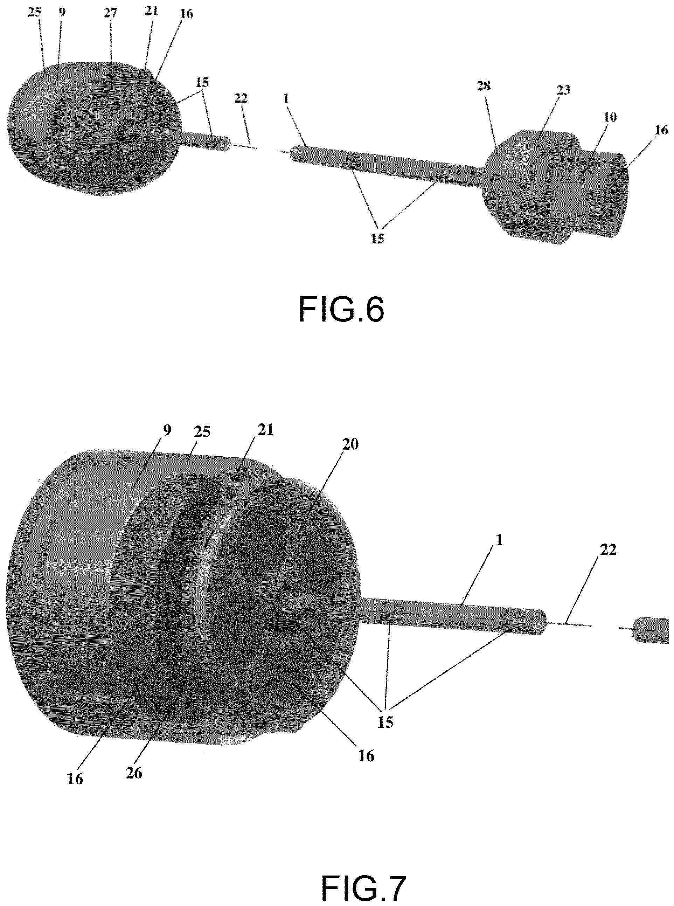

For sewing onto skin, housing 20 has eyes 21 (figure 7) equally spaced around the circumference of housing 20.

On the other end of broach 1, terminal half coupling 10 with permanent magnets 16 is press fitted/glued on flexible transmitting shaft 22 (figure 6).

Terminal half coupling 10 in the form of bushing is inside hermetic housing 23 with adaptor cover 28 connected to broach 1 and in contact with the internal driven half coupling formed by magnets 16 in the mount of second-stage impeller 3.

By way of the driving half coupling of the power unit consisting of pulley 26 and permanent magnets 16, the torque motion is transmitted to terminal half coupling 18 located inside the patient's body via a permanent magnetic field. Then, the rotating torque from first terminal half coupling 18 passes on to connected flexible transmitting shaft 22 inside broach 1 placed inside a vessel up to the ascending aorta that contains the pump unit of the microaxial pump. At the end of flexible transmitting shaft 22 in hermetic housing 23 there is terminal half coupling 180 that transmits rotating torque through the wall of hermetic housing 23 on to the internal driven half coupling in the mount of second-stage impeller 3.

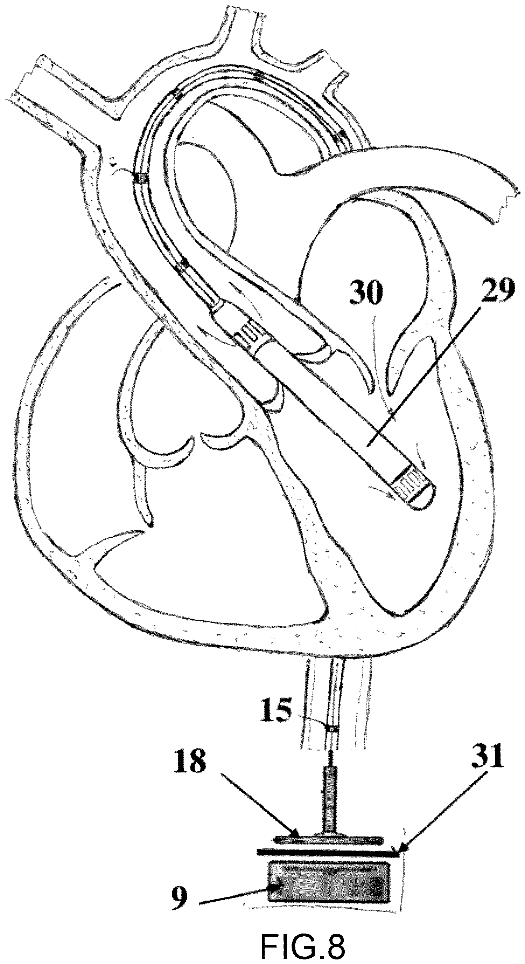

The microaxial pump operates like this (figure 8).

The microaxial pump is placed in the cavity of the left ventricle by one of the known means:
- via a vascular implant anastomosed with the ascending aorta and farther on via the aortic valve to the left ventricle cavity;
- via a vascular implant anastomosed with the clavicular artery on the left;
- via the femoral artery and the ascending aorta.

Placing the pump inside the right ventricle is possible antegradedly via the femoral vein and the right atrium.

After the pump starts working, blood is taken from the cavity of the left or right ventricle by rotating impellers via a terminal broach. Passing through a flexible insert, blood gets to the inflow straightener, which thanks to vane positioning provides almost impactless flow onto the vanes of the first-stage impeller. In the zone of the first-stage impeller, the flow receives additional kinetic energy from rotation of the impeller and passes on to the vanes of the first-stage straightener that serve to reduce the whirl in the flow after the rotor and to increase head.

Further on, the liquid flow gets to the zone of the second-stage impeller where it gets additional kinetic energy from shaft rotation and comes to the second-stage straightener with vanes that serve to reduce the whirl in the flow and to increase head. After the second-stage straightener the flow is discharged into the cavity of ascending aorta and gets to the larger blood circle ensuring the physiologically necessary blood discharge and pressure difference.

Rotation of impellers press fitted or glued on to the shaft is effected through the magnetic half coupling in the mount of the second-stage impeller. The mount of the second-stage impeller contains at least four permanent magnets. The rotating torque is transmitted via a permanent magnetic field from the second half coupling located inside a hermetic housing and installed either directly on the main shaft (with internal location of the motor), or press fitted/glued on the flexible shaft passing through the external flexible tube of the broach and sleeve/roller bearings set at intervals. On the other side of the external flexible tube of the broach, the flexible shaft has an internal magnetic half coupling that is press fitted/ glued on it. The internal magnetic half coupling is set in motion by a permanent magnetic field from the external magnetic half coupling installed on the shaft of the external motor connected to the control board that selects operation modes.

The use of the invention allows pumping blood without significantly impacting its temperature or shearing as the movable parts that come in contact with blood do not produce high gradients of shearing stress and the motor elements do not change the temperature in the areas of contact with the fluid.

The above description of an approximate variant of embodiment provides the general notion about principles of design, operation, manufacture and application of the device proposed by the invention. At least one of the variants of embodiment is illustrated in the attached drawings. Experts in the pertinent technical field believe that exact devices described in this document and illustrated in the drawings represent nonlimiting approximate variants of its embodiment and the scope of the present invention is determined exclusively by the formula of the invention. Characteristics illustrated or described in connection with one approximate variant of embodiment may be combined with characteristics of other embodiments. Such modifications and alterations are considered to be within the scope of the present invention.

## Claims

1. A blood pumping device with a noncontact magnetic coupling comprising located at least partially in gaps of large vessels:
a multi-stage pump unit having a housing, a spacer shaft located inside said housing, at least two axial stages containing impellers with curved vanes, placed on the spacer shaft, and a stator placed on the spacer shaft;
a power unit having a hermetic housing, a motor, located inside said housing, and a motor-driven main shaft;
a magnetic coupling having an external driving half coupling set on the main shaft of the power unit, and an internal driven half coupling located of an end side of a mount of the impeller of the second axial -stage of the multi-stage pump unit,
wherein the power unit is for transmitting rotation torque to the spacer shaft of the pump unit from the motor via a magnetic field.

2. The blood pumping device according to claim, 1 wherein the stator has at least three straighteners and one of them being an inflow straightener.

3. The blood pumping device according to claim 2, wherein the number of the curved vanes of at least one impeller differs from the number of vanes of at least one straightener of the stator.

4. The blood pumping device according to claim 2, wherein the vanes of the inflow straightener are aligned along an axis of the spacer shaft and/or at an angle to said axis.

5. The blood pumping device according to claim 2, wherein the spacer shaft is located into at least one pass-through and at least one terminal bearing support.

6. The blood pumping device according to claim 4, wherein bearings are sleeve and/or roller bearings.

7. The blood pumping device according to claim 1, wherein the curved vanes of the impellers of the multi-stage pump unit have the same or different profiles.

8. The blood pumping device according to claim 2, wherein an external diameter of the housing of the multi-stage pump unit is smaller than an diameter of large blood vessels.

9. The blood pumping device according to claim 1, wherein the external driving half coupling made in the form of a bushing with permanent magnets in its mount and the internal driven half coupling made in the form of permanent magnets imbedded in the mount of the impeller of second axial - stage of the multi-stage pump unit.

10. A blood pumping device with a noncontact magnetic coupling comprising:
a multi-stage pump unit located at least partially inside large vessels and having a housing, a spacer shaft, located inside said housing, and at least two axial stages placed on said spacer shaft and containing impellers with curved vanes and a stator;
a power unit located outside the patient's body and having a housing, a motor, located inside said housing, and a motor-driven main shaft,
wherein the main shaft transmits the torque to the spacer shaft of the pump unit by a flexible transmission shaft and a magnetic coupling via a magnetic field;
wherein the magnetic coupling having an external driving half coupling set on the main shaft of the power unit, and an internal driven half coupling located of an end side of a mount of the impeller of the second axial stage of the multi-stage pump unit,
wherein the flexible transmission shaft located inside the patient's body and having terminal magnetic half couplings fixed on each end:
a first terminal half coupling has a magnetic contact with the internal driven half coupling of the multi-stage pump unit,
a second terminal half coupling has a magnetic contact with the external driving half coupling of the power unit.

11. The blood pumping device according to claim 10, which additionally comprises a hollow flexible broach containing inside the flexible transmission shaft sitting on sleeve and/or roller bearings at equal or varied intervals along an axis of the flexible transmission shaft.

12. The blood pumping device according to claim 10, wherein the flexible transmission shaft made in the form of a steel string of round cross section.

13. The blood pumping device according to claim 10, wherein the external driving half coupling made in the form of a pulley with permanent magnets integrated inside it and the internal driven half coupling made in the form of permanent magnets imbedded in the mount of the impeller of second axial -stage of the multi-stage pump unit.

14. The blood pumping device according to claim 11, wherein the first terminal half coupling made in the form of a pulley with permanent magnets and the second terminal half coupling made in the form of a bushing with permanent magnets integrated in its mount.

15. The blood pumping device according to claim 14, wherein the pulley of the first terminal half coupling and the bushing of the second terminal half coupling are located in respective hermetic housings with an adaptor cover rigidly connected to the hollow flexible broach.
